Europäisches Patentamt

European Patent Office

Office européen des brevets

(19)

(11) EP 0 574 839 B1

# (12) EUROPÄISCHE PATENTSCHRIFT

(45) Veröffentlichungstag und Bekanntmachung des Hinweises auf die Patenterteilung:
**09.10.1996 Patentblatt 1996/41**

(51) Int Cl.6: **C07C 41/01**, C07C 67/00, C07C 43/12, C07C 43/225

(21) Anmeldenummer: **93109346.2**

(22) Anmeldetag: **11.06.1993**

(54) **Herstellung von Difluormethylethern und -estern**

Process for the preparation of difluormethylethers and esters

Préparation d'éthers ou d'esters du difluorméthyl

(84) Benannte Vertragsstaaten:
**AT BE CH DE DK ES FR GB GR IE IT LI LU NL PT SE**

(30) Priorität: **17.06.1992 DE 4219811**

(43) Veröffentlichungstag der Anmeldung:
**22.12.1993 Patentblatt 1993/51**

(73) Patentinhaber: **Solvay Fluor und Derivate GmbH D-30173 Hannover (DE)**

(72) Erfinder:
- **Naumann, Dieter**
  **D-4600 Dortmund 41 (DE)**
- **Tyrra, Wieland**
  **D-5042 Erftstadt-Liblar (DE)**
- **Möckel, Regina**
  **D-5000 Köln 51 (DE)**

(74) Vertreter: **Lauer, Dieter, Dr.**
**Solvay Pharma Deutschland GmbH**
**Hans-Böckler-Allee 20**
**30173 Hannover (DE)**

(56) Entgegenhaltungen:
**EP-A- 0 291 860**

- **CHEMICAL ABSTRACTS, vol. 103, no. 19, 11. November 1985, Columbus, Ohio, US; abstract no. 160622c, LANGE HORST ET AL. '(CF3)2Cd.D and (CF3)2Zn.D: new reagents for Difluorocarbene reactions.' Seite 724 ;Spalte 1 ;**
- **CHEMICAL ABSTRACTS, vol. 110, no. 13, 27. März 1989, Columbus, Ohio, US; abstract no. 114999c, HARTGRAVES, GREG A. ET AL. 'The preparation and allylation of (difluoromethyl)cadmium.' Seite 710 ;Spalte 1 ;**
- **JOURNAL OF FLUORINE CHEMISTRY., Bd.44, Nr.3, September 1989, LAUSANNE CH Seiten 433 - 440 QING-YUN CHEN ET AL. 'A simple convenient method for preparation of difluoromethyl ethers...'**
- **JOURNAL OF FLUORINE CHEMISTRY., Bd.26, Nr.1, September 1984, LAUSANNE CH Seiten 1 - 18 HORST LANGE ET AL. 'Bis(Perfluororgano) Cadmium-Verbindungen: Ein einfaches Darstellungsverfahren'**
- **DITTUS G. ET AL. 'Houben-Weyl - Methoden der organischen Chemie - Band VI /3' 1965 , THIEME VERLAG , STUTTGART * Seite 119 - Seite 121 ***
- **HANS-G. BOIT 'Beilsteins Handbuch der organischen Chemie - Band 6/2' 1978 , SPRINGER -VERLAG , BERLIN.HEIDELBERG.NEW YORK * Seite 611 ***

**Beschreibung**

Die Erfindung bezieht sich auf ein Verfahren zur Herstellung von Ether- und Esterverbindungen, welche eine Difluormethyl-Gruppe aufweisen, sowie auf neue, eine Difluormethyl-Gruppe enthaltende, nach dem erfindungsgemäßen Verfahren erhältliche Etherverbindungen.

Ether- und Esterverbindungen, die eine Difluormethyl-($CF_2H$-) Gruppe aufweisen, sind, in Kombination mit oberflächenaktiven Verbindungen, z.B. Phosphorsäureestern, zur Wasserentfernung von Oberflächen von Gegenständen verwendbar. In Kombination mit schmierend wirkenden Agenzien sind sie als Schmiermittel brauchbar. Ferner sind Difluormethylether, z.B. $CHF_2OC_6H_5$, $CHF_2OCH_2CF_3$, $CH_3CH_2OCF_2H$ oder $CH_3OCF_2H$, als Ersatzstoffe für FCKW verwendbar.

Difluormethylester sind Zwischenprodukte und als Lösungsmittel brauchbar.

Die Herstellung durch Fluorierung von entsprechenden Dichlormethylethern ist aufgrund der geringen Reaktivität dieser Verbindungen gegenüber einem Chlor/Fluor-Austausch (bei dem überdies noch ein Bindungsbruch am Sauerstoff-Atom erfolgen kann) sehr schwierig.

Die Herstellung von Difluormethylphenylethern aus Chloroform, Kaliumfluorid und Phenol, die von B.R. Langlois in Tetrahedron Lett. 32 (1991), Seiten 3691 - 3694 beschrieben wird, gelingt nur mit geringer Ausbeute.

Die Herstellung von Difluormethylethern, z. B. von Enfluorane oder Isofluorane, erfolgt durch Umsetzung der instabilen Ausgangsverbindung Fluorosulfonyldifluoroacetat und Alkoholen, siehe Chen und Wu, J. Fluorine Chem. 44 (1989), Seiten 433 bis 440. Die Autoren erwähnen, daß die Herstellung von Difluormethylethern auch aus Difluoraziridin und Alkohol in Glasampullen möglich ist. Mangels Beschaffbarkeit des Aziridins ist die Anwendbarkeit dieser Methode begrenzt.

Aufgabe der vorliegenden Erfindung ist es, eine neues Verfahren zur Herstellung von Difluormethylethern und -Estern anzugeben.

Diese Aufgabe wird durch das Verfahren der vorliegenden Erfindung und die neuen, bei dem erfindungsgemäßen Verfahren erhältlichen Difluormethylether gelöst.

Das erfindungsgemäße Verfahren zur Herstellung von Verbindungen der allgemeinen Formel (I)

$$HF_2C\text{-}O\text{-}R \qquad\qquad (I)$$

worin R für $R^1$, für ($R^1$-$R^2$-X) oder $R^1C(O)$ steht und $R^1$ lineares oder verzweigtes Alkyl mit 1 bis 20 C-Atomen; durch Alkoxy, substituierte Alkoxy, Halogen, CN, $NO_2$ substituiertes lineares oder verzweigtes Alkyl mit 1 bis 20 C-Atomen; Aryl; durch ein oder mehrere Substituenten ausgewählt aus Halogen, CN, $NO_2$, $C_1$-$C_6$-Alkyl, substituiertes Aryl; Aryl, substituiert durch eine oder mehrere Aryl- oder $C_1$-$C_6$-Alkyl-gruppen, wobei diese Aryl- oder Alkylgruppen ihrerseits durch eine oder mehrere Substituenten ausgewählt aus der Gruppe Halogen, CN, $NO_2$ substituiert sind; bedeutet

$R^1$ und $R^2$ gemeinsam eine Alkylenkette mit 2 bis 20 C-Atomen; eine durch Halogen, CN, $NO_2$, $C_1$-$C_6$-Alkyl, Aryl substituierte Alkylenkette mit 2 bis 20 C-Atomen; eine Alkylenkette mit 2 bis 20 C-Atomen, substituiert durch Aryl oder $C_1$-$C_6$-Alkyl, wobei diese Aryl- oder $C_1$-$C_6$-Alkylgruppen ihrerseits druch einen oder mehrere Substituenten ausgewählt aus der Gruppe Halogen, CN, $NO_2$ substituiert sind, bedeuten und

X Halogen, insbesondere Chlorid oder Fluorid bedeutet,

indem man Verbindungen der allgemeinen Formel (II) einsetzt

$$R^2\text{-}O\text{-}R^1 \qquad\qquad (II)$$

worin $R^1$ die oben bezeichnete Bedeutung besitzt, $R^2$ Wasserstoff, Alkyl mit 1 bis 20 C-Atomen, gegebenenfalls durch Alkoxy, substituiertes Alkoxy, Halogen, CN, $NO_2$ substituiertes Alkyl mit 1 bis 20 C-Atomen, bedeutet oder

$R^1$ und $R^2$ gemeinsam für die oben bezeichnete Alkylenkette stehen oder

indem man zur Herstellung von Estern, die die $CF_2H$-O-Gruppe aufweisen, Verbindungen der Formel (III)

$$R^2\text{-O-C(O)}R^1 \qquad\qquad \text{(III)}$$

worin $R^2$ Wasserstoff bedeutet und $R^1$ die obenbezeichnete Bedeutung aufweist,
in Gegenwart einer Lewissäure umsetzt mit einer die $CF_3$-Gruppe enthaltenden Cadmium-, Zink- oder Wismutverbindung ausgewählt aus der Gruppe umfassend $Cd(CF_3)_2$, $Zn(CF_3)_2$, $Bi(CF_3)_3$, $CdHal(CF_3)$, $ZnHal(CF_3)$, $BiHal(CF_3)_2$ und $BiHal_2(CF_3)$, worin Hal Halogenid, vorzugsweise Bromid oder Chlorid, bedeutet, welches in einem komplexierenden, aprotischen organischen Lösungsmittel gelöst ist, und das gebildete Reaktionsprodukt hydrolysiert.

"Alkoxy" bedeutet vorzugsweise $C_1$-$C_4$-Alkoxy, "substituiertes Alkoxy" bedeutet vorzugsweise durch Halogen, insbesondere Fluor ein- oder mehrfach substituiertes $C_1$-$C_4$-Alkoxy.

Gemäß einer Ausführungsform wird somit die Gruppe $R^2$ abgespalten. In diesem Fall ist $R^2$ vorzugsweise C1-C5-Alkyl, insbesondere Methyl, Ethyl oder t-Butyl.

$R^1$ ist vorzugsweise C1-C6-Alkyl oder Phenyl, oder durch Halogen, insbesondere ein oder mehrere Chlor- und/oder Fluoratome, substituiertes C1-C6-Alkyl oder derart substituiertes Phenyl. $R^1$ kann beispielsweise sein: Methyl, Ethyl, Phenyl, Fluormethyl, Difluorchlormethyl, Trifluormethyl, 2.2.2-Trifluorethyl, Pentafluorphenyl.

Gemäß einer weiteren Ausführungsform steht $R^1$ in den Verbindungen der allgemeinen Formel (I) für durch C1-C2-Alkoxy substituiertes C1-C2-Alkylen oder für A-O-B-O-C, worin A und B für C1-C2-Alkylen und C für C1-C4-Alkyl steht. Gemäß dieser Variante steht $R^1$ insbesondere für oligomere Reste $(OCH_2CH_2)_n$-OY mit n=1 bis 4, z. B. $CH_2CH_2$-O-$CH_2CH_2$-OY, worin Y für Methyl, Ethyl, Propyl und Butyl steht.

Gemäß einer weiteren Ausführungsform bilden $R^1$ und $R^2$ eine Alkylenkette mit 2 bis 4 C-Atomen oder eine durch ein oder mehrere Halogenatome, insbesondere Chlor und/oder Fluor substituierte Alkylenkette mit 2 bis 4C-Atomen.

Es sind verschiedenste Lewissäuren anwendbar. Geeignet sind beispielsweise Halogenverbindungen von Elementen der dritten und vierten Hauptgruppe. Besonders gut geeignet sind Halogenverbindungen von Elementen der dritten Hauptgruppe, Bor, Aluminium, Indium, und Gallium, insbesondere Chloride oder Fluoride. Sehr gut geeignet sind Halogenverbindungen von Bor und Indium. Sehr gut geeignet sind Bortrichlorid, Indiumtrichlorid, Bortrifluorid sowie Addukte von Bortrifluorid, insbesondere mit Nitrilen, wie Acetonitril, Ethern wie Dimethylether, Diethylether, Methyl-t-butylether. Auch Borsäureester sind geeignet.

Die Zink-, Wismut- oder Cadmiumverbindung wird gelöst in einem organischen Lösungsmittel eingesetzt. Sie liegt in Form von Addukten in dem Lösungsmittel vor. Hal kann neben Bromid und Chlorid, die bevorzugt sind, auch Jodid bedeuten.

Für die Erzeugung dieser Lösung kann der Fachmann verschiedene Methoden anwenden, wie anhand von Cadmiumverbindungen erläutert wird. Zink- und Wismutverbindungen behandelt man analog.

So kann man beispielsweise Cadmiumverbindungen der allgemeinen Formel $(CF_3)_2Cd\cdot D$ in einem entsprechenden Lösungsmittel auflösen. In diesen Verbindungen liegt die Cadmiumverbindung bereits als Addukt vor. Verbindungen dieses Typs sind bekannt, vgl. H. Lange, D. Naumann, J. Fluor.Chem., 26 (1984), Seiten 1 bis 18. Das Symbol "D" steht für organische Lewis-Basen. Es steht für 2 Moleküle von solchen Lewis-Basen, die nur ein Elektronenpaar koordinativ zur Verfügung stellen können, oder für 1 Molekül solcher Lewis-Basen, die zwei oder mehr Elektronenpaare koordinativ zur Verfügung stellen können. Zu den ersteren gehören beispielsweise Nitrile, wie Acetonitril, cyklische Amine, wie Pyridin, zu den letzteren gehören Ether, beispielsweise oligomere Ether, vorzugsweise der Formel $CH_3$-$(OCH_2CH_2)_n$-$OCH_3$ mit n=1 bis 4, wie Ethylenglycoldimethylether ("glyme"), Diethylenglycoldimethylether ("diglyme"), N,N,N',N'-Tetramethylethylendiamin ("TMED"). Diese Cadmiumverbindungen sind gegenüber Luftsauerstoff unempfindlich und bei Raumtemperatur stabile, kristalline Substanzen. Sie können, wie in der zitierten Literaturstelle angegeben, aus Dimethylcadmium, Diethylcadmium, Trifluormethyljodid und der entsprechenden Lewis-Base sowie dem anschließenden Abdampfen flüchtiger Verbindungen hergestellt werden. Die Herstellung der benötigten Dialkylcadmium-Verbindungen wird beschrieben in "Methoden der organischen Chemie (Houben-Weyl), 4. Auflage (1973), Band XIII/2a, Seiten 867 und 868". Die erhaltenen kristallinen Trifluormethylcadmium-Verbindungen können dann, wie oben schon gesagt, in einem komplexierenden Lösungsmittel gelöst werden. Diese Ausführungsform zur Erzeugung der Lösung ist bevorzugt.

Gemäß einer anderen Ausführungsform kann diese Lösung der Trifluormethylcadmium-Verbindung erzeugt werden, indem man, wie in der zitierten Literaturstelle beschrieben, die Trifluormethylcadmium-Verbindung in einer Lösung herstellt, die komplexierendes Lösungsmittel enthält, und in situ in dem erfindungsgemäßen Verfahren einsetzt.

Für den Fachmann ist klar, daß das komplexierende Lösungsmittel die Trifluoromethylcadmium-Verbindung stabilisiert. Es ist vorteilhaft, wenn während der Umsetzung immer eine zur Stabilisierung ausreichende Menge an komplexierendem Lösungsmittel in der Reaktionsmischung vorhanden ist. Für den Fachmann ist aber auch klar, daß das Lösungsmittel nicht aus diesem komplexierenden Lösungsmittel bestehen muß. Es können gewünschtenfalls auch nichtkomplexierende inerte, mit dem komplexierenden Lösungmittel mischbare organische Lösungsmittel in der Reaktionsmischung vorhanden sein können. Hierzu gehören beispielsweise Halogenkohlenwasserstoffe wie Chloroform, Kohlenwasserstoffe wie Pentan, Hexan oder Petrolether-Fraktionen, oder aromatische Lösungsmittel wie Toluol. Der Anteil solcher nicht komplexierend wirkender Lösungsmittel im Lösungsmittelgemisch kann bis zu 100 Gew.-% betragen, beispielsweise zwischen 0 und 90 Gew.-%.

Als komplexierendes Lösungsmittel verwendet man aprotische, polare organische Lewisbase-Verbindungen, die 1, 2 oder mehr Elektronenpaare zur koordinativen Bindung zur Verfügung stellen können. Hierzu gehören beispielsweise aliphatische Ether, z.B. Dialkylether wie Diethylether, cykloaliphatische Ether, beispielsweise Tetrahydrofuran, oligomere aliphatische Ether, beispielsweise die weiter oben erwähnten "glyme" und "diglyme", Nitrile, beispielsweise Acetonitril, oligomere tetraalkylsubstituierte Di- oder Polyamine, bei spielsweise Tetramethylethylendiamin, gemischte aliphatisch-aromatische Ether (z.B. Anisol), Lactame, Formamide, Carbonsäureamide, Sulfone.

Sofern man Ether-Verbindungen als Lösungsmittel verwenden will, ist es zweckmäßig, die in die Reaktion eingesetzten Ausgangsverbindungen der Formel (II), die ja Ether-Verbindungen sind, als Lösungsmittel einzusetzen.

Die Herstellung von $Zn(CF_3)_2 \cdot 2D$ beschreiben H. Lange und D. Naumann in J. Fluorine Chem. 26 (1984), Seiten 435 bzw. 444. Dabei wird $CF_3I$ mit Dialkylzink-Verbindungen, z.B. Dimethylzink oder Diethylzink, in Anwesenheit einer Lewis-Base, z.B. Glyme oder Diglyme, Pyridin, unter Bildung von $Zn(CF_3)_2 \cdot 2D$ umgesetzt.

Die Herstellung von $Bi(CF_3)_3$ beschreiben D. Naumann und W. Tyrra in J. Organomet. Chem. 334 (1987), Seiten 323 ff.

Die Herstellung von Trifluormethyl-Zink-, -cadmium- und -wismuthalogeniden wird in der EP-A-0 291 860 beschrieben. Bei diesem Verfahren wird Zink, Cadmium oder Wismut in metallischer Form in einem komplexierenden Lösungsmittel, beispielsweise Acetonitril, mit $CF_3Hal$, z. B. $CF_3Br$, umgesetzt. Die Reaktion kann durch Katalysatoren, z. B. Jod, sowie durch Ultraschall gefördert werden.

Die Umsetzung der Zink-, Wismut- oder Cadmiumverbindung mit der jeweils eingesetzten Ausgangsverbindung erfolgt bei einer Temperatur von -78 °C bis +20 °C.

Das Mol-Verhältnis der Ausgangsverbindung (II) zur eingesetzten Trifluormethylcadmium-Verbindung kann im Bereich von etwa 1 zu 1 bis 20:1 liegen. Es liegt vorzugsweise zwischen etwa 2:1 und 4:1. Der Einsatz von (II) als Lösungsmittel ist vorteilhaft.

Zweckmäßigerweise geht man so vor, daß man zunächst eine Mischung der Trifluormethylmetall-Verbindung und der Ausgangsverbindung der Formel (I) im organischen Lösungsmittel erzeugt und dann die Lewissäure zugibt. Das Mol-Verhältnis von Metallverbindung zu Lewissäure beträgt zwischen etwa 1:1,5 und 1:5. Es liegt vorzugsweise zwischen etwa 1:1,5 und 1:3.

Die Lewissäure, beispielsweise ein Bortrihalogenid-Addukt oder Indiumhalogenid wie Indiumtrichlorid, kann als Feststoff zugegeben werden. Es ist natürlich auch möglich, eine Aufschlämmung oder Lösung der Lewissäure beispielsweise im verwendeten Lösungsmittel zuzugeben.

Nach der Zugabe setzt dann eine exotherme Reaktion ein.

Die Reaktionsmischung kann beispielsweise unter Überleiten eines Inertgases wie Stickstoff gewünschtenfalls noch einige Zeit, beispielsweise bis zu einigen Stunden, zur Vervollständigung der Umsetzung belassen werden. Anschließend wird die Reaktionsmischung mit einer molaren Menge einer protischen Verbindung, beispielsweise einer Säure, einem Alkohol oder, vorteilhafterweise, mit Wasser versetzt. Zur Aufarbeitung können flüchtige Bestandteile fraktioniert destilliert werden. Auf diese Weise kann die gewünschte Difluormethyl-Etherverbindung der allgemeinen Formel (I) isoliert werden.

Ohne daß eine Erklärung für den Reaktionsmechanismus gegeben werden soll, wird angenommen, daß die Bindung zwischen dem Sauerstoffatom und einem der beiden organischen Reste, beispielsweise $R^2$, gebrochen wird und dieser organische Rest schließlich durch die $CF_2H$-Gruppe ersetzt ist. Sofern ein Halogenid als Lewissäure verwendet wird, bildet sich in der Reaktionsmischung ein entsprechendes Halogenid dieses abgespaltenen organischen Restes $R^2$. Sofern nun als Ausgangsverbindung eine Verbindung der allgemeinen Formel (II) eingesetzt sind, in welcher $R^1$ und $R^2$ gemeinsam eine (verbrückende) Alkylenkette bilden, entsteht demgemäß eine Verbindung der allgemeinen Formel $HF_2C-O-R^1R^2X$, wenn als Lewissäure beispielsweise $AlX_3$, $InX_3$ oder $BX_3$ mit X=Cl, Br eingesetzt wurde.

Wie in Anspruch 1 ausgeführt, eignet sich das erfindungsgemäße Verfahren zur Herstellung von Verbindungen der allgemeinen Formel (I), in welcher R die oben angegebene Bedeutung besitzt. Besonders gut eignet sich das erfindungsgemäße Verfahren zur Herstellung von Verbindungen der allgemeinen Formel (I), in welcher R $CH_3$, $C_2H_5$, $CH_2CF_3$, $(CH_2)_3CH_3$, $(CH_2)_4Cl$, $C_6H_5$, $C_6F_5$ oder $CH_2CH_2-O-CH_2CH_2-OCH_3$ bedeutet.

Ein weiterer Gegenstand sind neue, als Schmiermittel brauchbare, nach dem erfindungsgemäßen Verfahren erhältliche Verbindungen der allgemeinen Formel $HF_2C-(OCH_2CH_2)_n-OY$ mit n = 2 bis 4 und Y = C1-C4-Alkyl, vorzugsweise Verbindungen der allgemeinen Formel (IV)

$$HF_2C-O-CH_2CH_2-OCH_2CH_2-OY \qquad\qquad (IV),$$

worin Y für Methyl, Ethyl, Propyl und Butyl steht. Neu ist auch die Verbindung $HF_2C-O-CH_2CH_2Cl$.

Die nach dem erfindungsgemäßen Verfahren erhältlichen Verbindungen sind für die eingangs genannten Zwecke verwendbar, für die diese üblicherweise verwendet werden, z.B. als Zwischenprodukte in der chemischen Synthese, als Schmiermittel, Kühlmittel etc..

Die folgenden Beispiele sollen die Erfindung weiter erläutern, ohne sie in ihrem Umfang einzuschränken.

Beispiel 1: Herstellung von $HF_2C-O-(CH_2)_4Cl$

Als Trifluormethylcadmium-Verbindung wurde das Addukt von Diethylenglycoldimethylether und Bis-trifluormethylcadmium, $Cd(CF_3)_2$ Diglyme, verwendet. Diese Verbindung wurde gemäß der Vorschrift in H. Lange, D. Naumann, J. Fluor. Chem. 26 (1984), Seite 13 aus Trifluormethyljodid, Diglyme und Dimethylcadmium hergestellt.

7,6 g (19,76 mmol) $Cd(CF_3)_2$ Diglyme wurden in Inertgasatmosphäre in 25 ml Tetrahydrofuran (THF) gelöst. In die mittels eines Magnetrührers gerührte Lösung wurden bei Raumtemperatur 2,9 g (13,11 mmol) Indiumtrichlorid zugegeben. Es setzte eine exotherme Reaktion ein, und ein weißer Feststoff fiel aus. Die Reaktionsmischung wurde noch 24 Stunden gerührt. Hierbei wurde die Suspension zunehmend zähflüssig.

Der Reaktionsmischung wurden dann 10 g Wasser zugesetzt. Zur Aufarbeitung wurde das Lösungsmittel Tetrahydrofuran sowie leicht flüchtige Bestandteile bei Raumtemperatur im Hochvakuum abgedampft.

Der Rückstand wurde sodann auf 50 °C erwärmt und im Hochvakuum fraktioniert destilliert.

Die Charakterisierung durch Kernresonanzspektroskopie und Massenspektroskopie ergab folgende Daten:

$HCF_2O(CH_2)_4Cl$:

$^1H$ n.m.r.: (p.p.m.)/$CDCl_3$: 6.12 (t,$^2J_{F-H}$ 75.2 Hz, $HCF_2$), 3.56 (m, $CH_2$), 1.66 (m, $CH_2$), 1.55 (m, $CH_2$), 3.50 (m, $CH_2$).

$^{19}F$ n.m.r.: (p.p.m.)/$CDCl_3$: -84.35 (d,$^2J_{F-H}$ 75.2 Hz).

$^{13}C$ n.m.r.: (p.p.m.)/$CDCl_3$: 116.06 (t,$^1J_{F-C}$ 260.1 Hz, $CF_2HO-$), 62.35 (t, $^3J_{F-C}$ 5.1 Hz, $CF_2HOCH_2-$), 26,05 ($-CH_2-$), 29,52 ($-CH_2-$), 44.95 ($-CH_2Cl$).

m.s. (70 eV; $^{35}Cl$ isotope; m/e): 158 (4.0%, M+),

$CF_2HO(CH_2)_2O(CH_2)_2OCH_3$:

$^1H$ n.m.r.: ($CF_2HO$) (p.p.m.)/$CDCl_3$: 6.20 (t,$^2J_{F-H}$ 75.2 Hz). ($CF_2HO$) (p.p.m.)/$CDCl_3$: -84.63

$^{19}F$ n.m.r.: (d,$^2J_{F-H}$ 74.6 Hz).

m.s. (70 ev; m>80; m/e): 170 (10.1%, M+).

Beispiel 2: Herstellung von Difluormethyl-2,2,2-trifluorethylether

4,77 g $Zn(CF_3)Br\cdot2CH_3CN$ (16,09 mmol), hergestellt wie in der EP-A 291 860 beschrieben, wurden in 40 ml $CH_2Cl_2$ suspendiert. Bei einer Temperatur von -78 °C wurde diese Suspension mit 2 g $BF_3\cdot CH_3CN$ (18,37 mmol) versetzt. Anschließend erfolgte die Zugabe von 2,5 ml Trifluorethanol (34,72 mmol). Das Reaktionsgemisch wurde eine Stunde bei - 78 °C gerührt, im folgenden auf -55 °C und innerhalb von ca. 3 Stunden auf Raumtemperatur erwärmt. Während dieser Zeit wurde $Zn(CF_3)Br\cdot2CH_3CN$ vollständig umgesetzt. Die Aufarbeitung des Reaktionsansatzes erfolgte destillativ im Vakuum. Die bei 0 °C erhaltene Fraktion enthielt neben Difluormethyl-2,2,2-trifluorethylether eine weitere Verbindung, bei der es sich um Bis-2,2,2-trifluorethylether ($^{19}F$-NMR: δ -74,63 ppm; $^2J(^{19}F-^1H)$ = 8,9 Hz) handeln kann (Integrationsverhältnis: 1,3:1). Die zweite Fraktion (0 °C bis RT) enthielt nur Spuren von Difluormethyl-2,2,2-trifluorethylether, während hier vermutlich $CF_3CH_2OCH_2CF_3$ als Hauptprodukt vorliegt (Integrationsverhältnis: 1:15).

$^{19}F$-NMR-Daten von $CHF_2OCH_2CF_3$:
($CH_2Cl_2$, 20 °C)

δ ($CF_3$) -75,36 ppm, tt
$^3J(^{19}F-^1H)$ = 8,3 Hz
$^5J(^{19}F-^{19}F)$ = 2,5 Hz
δ ($CHF_2$) -86,74 ppm, dq
$^2J(^{19}F-^1H)$ = 72,5 Hz
$^5J(^{19}F-^{19}F)$ = 2,5 Hz

Beispiel 3: Herstellung von Difluormethylphenylether

15,97 g $Zn(CF_3)Br\cdot2CH_3CN$ (53,87 mmol) wurden in 60 ml $CH_2Cl_2$ suspendiert. Die Suspension wurde unter Rühren bei -78 °C mit 6 g $BF_3\cdot CH_3CN$ (55,12 mmol) versetzt. Anschließend erfolgte die Zugabe von 10 g Phenol (106,26 mmol). Das Reaktionsgemisch wurde ca. 1 Stunde bei -78 °C gerührt, im folgenden auf -55 °C und während weiterer 3 Stunden auf Raumtemperatur erwärmt. $Zn(CF_3)Br \cdot 2CH_3CN$ war 19F-NMR-spektroskopisch nicht mehr nachweisbar. Die destillative Aufarbeitung erfolgte im Vakuum. Bei 0 °C wurde der größte Anteil des Lösungsmittels aus dem Reaktionsgemisch entfernt. Durch Destillation im Heißluftstrom wurde eine rotbraun gefärbte Lösung erhalten, die neben Difluormethylphenylether Phenol, Acetonitril, Dichlormethan, vermutlich $C_6H_5OCF_2OC_6H_5$ (19F-NMR: $\delta$ -76,39 ppm, $^1J(^{19}F-^{13}C) = 290,4$ Hz, $\Delta \delta$ 0,1282 ppm) und $BF_3$-Addukte enthielt. Nach Zugabe von NaF wurde das Gemisch im Vakuum (170 - 210 mbar) von restlichem Lösungsmittel befreit. Die weitere Aufarbeitung erfolgte durch erneute Destillation im Heißluftstrom, wodurch eine hohe Anreicherung von Difluormethylphenylether erzielt wurde.

19F-NMR-Daten von $CHF_2OC_6F_5$

($CDCl_3$, 20 °C)

$\delta$ ($CHF_2$) -81,23 ppm, d
$^2J(^{19}F-^1H) = 73,8$ Hz
$^1J(^{19}F-^{13}C) = 258,9$ Hz; $\Delta \delta$ 0,1247 ppm

1H-NMR-Daten von $CHF_2OC_6H_5$

($CDCl_3$, 20 °C)

$\delta$ ($CHF_2$) 6,50 ppm, t
$^2J(^{19}F-^1H) = 74,0$ Hz
$\delta$ (Aromat) 6,85; 7,12; 7,35 ppm

Beispiel 4: Herstellung von Chlordifluoressigsäuredifluormethylester

1,07 g $Zn(CF_3)Br \cdot 2CH_3CN$ (3,61 mmol) wurden in 10 ml $CH_2Cl_2$ suspendiert und bei -65 °C unter Rühren mit 0,4 g $BF_3 \cdot CH_3CN$ (3,67 mmol) versetzt. Anschließend erfolgte die Zugabe von ca. 1,04 g Chlordifluoressigsäure (ca. 7,97 mmol). Nach 30 Minuten wurde das Kältebad entfernt und die Mischung unter Rühren auf Raumtemperatur erwärmt. Die eingesetzte Zn-Verbindung war nicht mehr nachweisbar.

19F-NMR-Daten von $CF_2ClCOOCF_2H$:

($CH_2Cl_2$, 20 °C)

$\delta$ ($CF_2Cl$) -65,91 ppm
$\delta$ ($CF_2H$) -91,74 ppm, d
$^2J(^{19}F-^1H) = 68,7$ Hz

Beispiel 5: Herstellung von Trifluoressigsäuredifluormethylester

1,12 g $Zn(CF_3)Br \cdot 2CH_3CN$ (3,78 mmol) wurden in 10 ml $CH_2Cl_2$ suspendiert und bei -65 °C mit 0,4 g $BF_3 \cdot CH_3CN$ (3,67 mmol) versetzt. Unter Rühren erfolgte die Zugabe von 0,7 ml Trifluoressigsäure (9,09 mmol). Nach 30 Minuten wurde das Kältebad entfernt und die Reaktionsmischung unter Rühren auf Raumtemperatur erwärmt.

19F-NMR-Daten von $CF_3COOCF_2H$:

($CH_2Cl_2$, 20 °C)

$\delta$ ($CF_3$) -76,1 ppm
$\delta$ ($CF_2H$) -91,93 ppm, d
$^2J(^{19}F-^1H) = 64,8$ Hz

Beispiel 6: Herstellung von Pentafluorbenzoesäuredifluormethylester

3 g $Zn(CF_3)Br \cdot 2CH_3CN$ (10,12 mmol) wurden in 20 ml $CH_2Cl_2$ suspendiert. Das Gemisch wurde auf -60 °C gekühlt und unter Rühren mit 1,1 g $BF_3 \cdot CH_3CN$ (10,10 mmol) versetzt. Anschließend erfolgte die Zugabe von 4,3 g Pentafluorbenzoesäure (20,27 mmol). Das Reaktionsgemisch wurde 1 Stunde bei dieser Temperatur belassen, bevor das Kältebad entfernt wurde. Unter Rühren erwärmte sie sich auf Raumtemperatur. $Zn(CF_3)Br \cdot 2CH_3CN$ war vollständig umgesetzt.

19F-NMR-Daten von $C_6F_5COOCF_2H$

($CH_2Cl_2$, 20 °C)

$\delta$ ($CF_2H$) -92,54 ppm, d

$^2J(^{19}F\text{-}^1H) = 70{,}1$ Hz
$\delta$ (C$_6$F$_5$, o) -136,87 ppm
$\delta$ (C$_6$F$_5$, p) -145,66 ppm
$\delta$ (C$_6$F$_5$, m) -160,70 ppm

**Patentansprüche**

1. Verfahren zur Herstellung von Verbindungen der allgemeinen Formel (I)

$$\text{HF}_2\text{C-O-R} \qquad \text{(I)}$$

worin R für R$^1$, R$^1$C(O) oder für (R$^1$-R$^2$-X) steht und R$^1$ lineares oder verzweigtes Alkyl mit 1 bis 20 C-Atomen; durch Alkoxy, substituiertes Alkoxy, Halogen, CN, NO$_2$ substituiertes lineares oder verzweigtes Alkyl mit 1 bis 20 C-Atomen; Aryl; durch ein oder mehrere Substituent ausgewählt aus Halogen, CN, NO$_2$, C$_1$-C$_6$-Alkyl substituiertes Aryl; Aryl, substituiert durch eine oder mehrere Aryl- oder C$_1$-C$_6$-Alkylgruppen, wobei diese Aryl- oder Alkylgruppen ihrerseits durch einen oder mehrere Substituenten ausgewählt aus der Gruppe Halogen, CN, NO$_2$ substituiert sind; bedeutet

R$^1$ und R$^2$ gemeinsam eine Alkylenkette mit 2 bis 20 C-Atomen; eine durch Halogen, CN, NO$_2$, C$_1$-C$_6$-Alkyl, Aryl substituierte Alkylenkette mit 2 bis 20 C-Atomen; eine Alkylenkette mit 2 bis 20 C-Atomen, substituiert durch Aryl oder C$_1$-C$_6$-Alkyl, wobei diese Aryl- oder C$_1$-C$_6$-Alkylgruppen ihrerseits durch einen oder mehrere Substituenten ausgewählt aus der Gruppe Halogen, CN, NO$_2$ substituiert sind, bedeuten und

X Halogen, insbesondere Chlorid oder Fluorid bedeutet,

indem man Verbindungen der allgemeinen Formel (II)

$$\text{R}^2\text{-O-R}^1 \qquad \text{(II)}$$

worin R$^1$ die oben bezeichnete Bedeutung besitzt, R$^2$ Wasserstoff, Alkyl mit 1 bis 20 C-Atomen, gegebenenfalls durch Alkoxy, substituiertes Alkoxy, Halogen, CN, NO$_2$ substituiertes Alkyl mit 1 bis 20 C-Atomen, bedeutet oder

R$^1$ und R$^2$ gemeinsam für die oben bezeichnete Alkylenkette stehen oder

indem man zur Herstellung von Estern, die die CF$_2$H-O-Gruppe aufweisen, Verbindungen der Formel (III)

$$\text{R}^2\text{-O-C(O)R}^1 \qquad \text{(III)}$$

worin R$^2$ Wasserstoff bedeutet und R$^1$ die obenbezeichnete Bedeutung aufweist,
in Gegenwart einer Lewissäure umsetzt mit einer die CF$_3$-Gruppe enthaltenden Cadmium-, Zink- oder Wismutverbindung ausgewählt aus der Gruppe umfassend Cd(CF$_3$)$_2$, Zn(CF$_3$)$_2$, Bi(CF$_3$)$_3$, CdHal(CF$_3$), ZnHal(CF$_3$), BiHal(CF$_3$)$_2$ und BiHal$_2$(CF$_3$), worin Hal Halogenid, vorzugsweise Bromid oder Chlorid, bedeutet, welches in einem organischen Lösungsmittel gelöst ist, und das gebildete Reaktionsprodukt hydrolysiert.

2. Verfahren nach Anspruch 1, dadurch gekennzeichnet, daß man als Lewissäure Halogenverbindungen von Elementen der dritten Hauptgruppe, insbesondere Chloride oder Fluoride, verwendet.

3. Verfahren nach Anspruch 2, dadurch gekennzeichnet, daß man Halogenverbindungen von Bor oder Indium, insbesondere Bortrifluorid, Bortrifluorid-Addukte mit Acetonitril, Dimethylether, Diethylether, Methyl-t-butylether, Bortrichlorid oder Indiumtrichlorid als Lewissäure verwendet.

4. Verfahren nach einem der vorhergehenden Ansprüche, dadurch gekennzeichnet, daß man als Lösungsmittel aliphatische Ether, cykloaliphatische Ether, oligomere aliphatische Ether, Nitrile, Lactame, Formamide, Carbonsäureamide, Sulfone, halogenierte Kohlenwasserstoffe, Alkohole, gegebenenfalls substituierte aromatische Kohlenwasserstoffe verwendet.

5. Verfahren nach Anspruch 4, dadurch gekennzeichnet, daß man als Lösungsmittel oligomere Ether, insbesondere oligomere Ether der allgemeinen Formel

$$CH_3\text{-}(OCH_2CH_2)_n\text{-}OCH_3,$$

worin n 1 bis 4 bedeutet, einsetzt.

6. Verfahren nach einem der vorhergehenden Ansprüche, dadurch gekennzeichnet, daß man die Umsetzung der Verbindung der allgemeinen Formel (II) zu Verbindungen der allgemeinen Formel (I) bei einer Temperatur von -78 °C bis +20 °C durchführt.

7. Verfahren nach einem der vorhergehenden Ansprüche, dadurch gekennzeichnet, das das Mol-Verhältnis der Verbindung der allgemeinen Formel (II) zur Zink-, Wismut- oder Cadmiumverbindung zwischen etwa 1:1 bis 20:1, vorzugsweise von 2:1 bis 4:1 beträgt.

8. Verfahren nach einem der vorhergehenden Ansprüche, dadurch gekennzeichnet, daß das Mol-Verhältnis von Lewissäure zu Zink-, Wismut- oder Cadmiumverbindung zwischen etwa 1,5:1 und 5:1, vorzugsweise zwischen etwa 1,5:1 und 3:1 liegt.

9. Verfahren nach einem der vorhergehenden Ansprüche, dadurch gekennzeichnet, daß $R^2$ eine C1-C5-Alkylgruppe, vorzugsweise Methyl, Ethyl oder t-Butyl ist.

10. Verfahren nach einem der vorhergehenden Ansprüche, dadurch gekennzeichnet, daß $R^1$ gegebenenfalls durch Halogen substituiertes C1-C6-Alkyl oder gegebenenfalls durch Halogen substituiertes Phenyl ist, oder daß $R^1$ und $R^2$ eine gegebenenfalls durch Halogen substituierte Alkylkette mit 2 bis 4 C-Atomen darstellen.

11. Verbindungen der Formel

$$HF_2C\text{-}(OCH_2CH_2)_n\text{-}OY$$

mit n = 2, 3 oder 4 und Y = C1-C4-Alkyl

12. 
$$HF_2C\text{-}O\text{-}CH_2CH_2\text{-}OCH_2CH_2\text{-}OY$$

mit Y = Methyl, Ethyl, Propyl, Butyl

13. 
$$HF_2C\text{-}O\text{-}CH_2CH_2Cl$$

## Claims

1. A process for the production of compounds of the general formula (I),

$$HF_2C\text{-}O\text{-}R \qquad\qquad (I)$$

wherein R stands for $R^1$, $R^1C(O)$ or for $(R^1\text{-}R^2\text{-}X)$ and $R^1$ is linear or branched alkyl with 1 to 20 C atoms; linear or branched alkyl with 1 to 20 C atoms substituted by alkoxy; substituted alkoxy, halogen, CN, $NO_2$; aryl; aryl, substituted by one or more substituents selected from halogen, CN, $NO_2$, C1 to C6 alkyl; aryl, substituted by one or more aryl groups or C1 to C6 alkyl groups, these aryl groups or alkyl groups being substituted by one or more substituents selected from the group of halogen, CN, $NO_2$;

$R^1$ and $R^2$ together form an alkylene chain with 2 to 20 C atoms; an alkylene chain with 2 to 20 C atoms substituted by halogen, CN, $NO_2$, C1 to C6 alkyl, or aryl; an alkylene chain with 2 to 20 C atoms, substituted by aryl or C1 to C6 alkyl, these aryl or C1 to C6 alkyl groups being substituted by one or more substituents selected from the group consisting of halogen, CN, $NO_2$; and

X is halogen, in particular chloride or fluoride,

in that compounds of the general formula (II) are used,

$$R^2\text{-O-}R^1 \tag{II}$$

wherein $R^1$ has the meaning given above;

$R^2$ is hydrogen, alkyl with 1 to 20 C atoms, alkyl with 1 to 20 C atoms substituted by alkoxy, substituted alkoxy, halogen, CN, $NO_2$, or

$R^1$ and $R^2$ together form an alkylene chain given above, or

in that in order to produce esters which bear the $CF_2$H-O group, compounds of formula (III) are used,

$$R^2\text{-O-C(O)}R^1 \tag{III}$$

wherein $R^2$ is hydrogen and $R^1$ has the above meaning,

are reacted in the presence of a Lewis acid with a cadmium, zinc or bismuth compound containing the $CF_3$ group and selected from the group comprising $Cd(CF_3)_2$, $Zn(CF_3)_2$, $Bi(CF_3)_3$, $CdHal(CF_3)$, $ZnHal(CF_3)$, $BiHal(CF_3)_2$ and $BiHal_2(CF_3)$, wherein Hal is halide, preferably bromide or chloride, which is dissolved in an organic solvent, and the resulting reaction product is hydrolysed.

2. A process according to Claim 1, characterised in that halogen compounds of elements of the third main group, in particular chlorides or fluorides, are used as Lewis acid.

3. A process according to Claim 2, characterised in that halogen compounds of boron or indium, in particular boron trifluoride, boron trifluoride adducts with acetonitrile, dimethyl ether, diethyl ether, methyl-t-butyl ether, boron trichloride or indium trichloride are used as Lewis acid.

4. A process according to one of the preceding Claims, characterised in that aliphatic ethers, cycloaliphatic ethers, oligomeric aliphatic ethers, nitriles, lactams, formamides, carboxylic acid amides, sulphones, halogenated hydrocarbons, alcohols and optionally substituted aromatic hydrocarbons are used as solvent.

5. A process according to Claim 4, characterised in that oligomeric ethers, in particular oligomeric ethers of the general formula

$$CH_3\text{-(O}CH_2CH_2)_n\text{-O}CH_3,$$

wherein n is 1 to 4, are used as solvents.

6. A process according to one of the preceding Claims, characterised in that the reaction of the compound of the general formula (II) to form compounds of the general formula (I) is carried out at a temperature of -78°C to +20°C.

7. A process according to one of the preceding Claims, characterised in that the molar ratio of the compound of the general formula (II) to the zinc, bismuth or cadmium compound is between about 1:1 and 20:1, preferably from 2:

1 to 4:1.

8. A process according to one of the preceding Claims, characterised in that the molar ratio of Lewis acid to zinc, bismuth or cadmium compound is between about 1.5:1 and 5:1, preferably between about 1.5:1 and 3:1.

9. A process according to one of the preceding Claims, characterised in that $R^2$ is a C1-C5-alkyl group, preferably methyl, ethyl or t-butyl.

10. A process according to one of the preceding Claims, characterised in that $R^1$ is C1-C6-alkyl optionally substituted by halogen, or phenyl optionally substituted by halogen, or that $R^1$ and $R^2$ represent an alkyl chain with 2 to 4 C atoms optionally substituted by halogen.

11. Compounds of the formula

$$HF_2C\text{-}(OCH_2CH_2)_n\text{-}OY$$

with n = 2, 3 or 4 and
Y = C1-C4-alkyl.

12.
$$HF_2C\text{-}O\text{-}CH_2CH_2\text{-}OCH_2CH_2\text{-}OY$$

with
Y = methyl, ethyl, propyl, butyl.

13.
$$HF_2C\text{-}O\text{-}CH_2CH_2Cl.$$

**Revendications**

1. Procédé de préparation de composés de formule générale (I)

$$HF_2C\text{-}O\text{-}R \qquad \qquad (I)$$

dans laquelle R représente $R^1$, ($R^1$ - $R^2$ - X) ou $R^1C(O)$ et où $R^1$ représente un alkyle linéaire ou ramifié ayant de 1 à 20 atomes de carbone; un alkyle linéaire ou ramifié ayant de 1 à 20 atomes de carbone substitué par un alcoxy, un alcoxy substitué, un halogène, CN, $NO_2$; un aryle; un aryle substitué par un ou plusieurs substituants choisis parmi les halogènes, CN, $NO_2$, les alkyle en $C_1$-$C_6$; un aryle substitué par un ou plusieurs groupes aryle ou alkyle en $C_1$-$C_6$ dans lesquels ces groupes aryle ou alkyle sont substitués à leur tour par un ou plusieurs substituants choisis dans le groupe des halogènes, de CN, de $NO_2$;
$R^1$ et $R^2$ représentent ensemble une chaîne alkylène ayant de 2 à 20 atomes de carbone, une chaîne alkylène ayant de 2 à 20 atomes de carbone substituée par un halogène, CN, $NO_2$, un alkyle en $C_1$-$C_6$, un aryle; une chaîne alkylène ayant de 2 à 20 atomes de carbone substituée par un aryle ou un alkyle en $C_1$-$C_6$ dans lequel ces groupes aryle ou alkyle en $C_1$-$C_6$ sont substitués à leur tour par un ou plusieurs substituants choisis dans le groupe formé par les halogènes, CN, $NO_2$; et
X représente un halogène et, en particulier, un chlorure ou un fluorure,

dans lequel des composés de formule générale (II)

$$R^2\text{-}O\text{-}R^1 \qquad \qquad (II)$$

dans laquelle $R^1$ a la signification mentionnée ci-dessus, $R^2$ représente un atome d'hydrogène, un alkyle ayant de 1 à 20 atomes de carbone, un alkyle ayant de 1 à 20 atomes de carbone substitué éventuellement par un alcoxy, un alcoxy substitué, un halogène, CN, NO$_2$, ou

R$^1$ et R$^2$ forment ensemble la chaîne alkylène mentionnée ci-dessous, ou

dans lequel, pour la préparation d'esters qui présentent le groupe CF$_2$H-O, des composés de formule (III)

$$R^2\text{-O-C(O)}R^1 \qquad\qquad (III)$$

dans laquelle R$^2$ représente un atome d'hydrogène et R$^1$ a la signification mentionnée ci-dessus,

sont mis à réagir en présence d'un acide de Lewis avec un composé de cadmium, de zinc ou de bismuth contenant le groupe CF$_3$, choisi dans le groupe comprenant Cd(CF$_3$)$_2$, Zn(CF$_3$)$_2$, Bi(CF$_3$)$_3$, CdHal(CF$_3$), ZnHal(CF$_3$), BiHal (CF$_3$)$_2$ et BiHal$_2$(CF$_3$), où Hal représente un halogénure et, de préférence, un bromure ou un chlorure, qui est dissous dans un solvant organique, avec hydrolyse du produit de réaction ainsi formé.

2. Procédé selon la revendication 1, caractérisé en ce que l'on utilise comme acide de Lewis, des composés halogénés des éléments du troisième groupe principal et, en particulier, des chlorures ou des fluorures.

3. Procédé selon la revendication 2, caractérisé en ce qu'on utilise comme acide de Lewis, des composés halogénés du bore ou de l'indium et, en particulier, du trifluorure de bore, des produits d'addition du trifluorure de bore avec l'acétonitrile, l'éther diméthylique, l'éther diéthylique, l'éther méthyl-t-butylique, le trichlorure de bore ou le trichlorure d'indium.

4. Procédé selon l'une des revendications précédentes, caractérisé en ce que l'on utilise comme solvant des éthers aliphatiques, des éthers cycloaliphatiques, des éthers aliphatiques oligomères, des nitriles, des lactames, le formamide, des amides d'acide carboxylique, des sulfones, des hydrocarbures halogénés, des alcools, des hydrocarbures aromatiques éventuellement substitués.

5. Procédé selon la revendication 4, caractérisé en ce que l'on utilise comme solvant des éthers oligomères et, en particulier, des éthers oligomères de formule générale

$$CH_3\text{-}(OCH_2CH_2)_n\text{-}OCH_3$$

dans laquelle n représente de 1 à 4.

6. Procédé selon l'une des revendications précédentes, caractérisé en ce que l'on effectue la transformation du composé de formule générale (II) en composé de formule générale (I) à une température entre -78°C et +20°C.

7. Procédé selon l'une des revendications précédentes, caractérisé en ce que le rapport molaire du composé de formule générale (II) au composé de zinc, de bismuth ou de cadmium, est compris entre environ 1:1 et 20:1, et de préférence entre 2:1 et 4:1.

8. Procédé selon l'une des revendications précédentes, caractérisé en ce que le rapport molaire de l'acide de Lewis au composé de zinc, de bismuth ou de cadmium est compris entre environ 1,5:1 et 5:1, et de préférence entre environ 1,5:1 et 3:1.

9. Procédé selon l'une des revendications précédentes, caractérisé en ce que R$^2$ est un groupe alkyle en C$_1$-C$_5$ et, de préférence, un groupe méthyle, éthyle ou t-butyle.

10. Procédé selon l'une des revendications précédentes, caractérisé en ce que R$^1$ est un alkyle en C$_1$-C$_6$, éventuellement substitué par un halogène, ou un phényle éventuellement substitué par un halogène, ou en ce que R$^1$ et R$^2$ représentent une chaîne alkyle ayant de 2 à 4 atomes de carbone, éventuellement substituée par un halogène.

11. Composés de formule

$$HF_2C\text{-}(OCH_2CH_2)_n\text{-}OY$$

avec n = 2, 3 ou 4 et Y = $C_1$-$C_4$-alkyle.

12. $$HF_2C\text{-}O\text{-}CH_2CH_2\text{-}OCH_2CH_2\text{-}OY$$

avec Y = méthyle, éthyle, propyle, butyle.

13. $$HF_2C\text{-}O\text{-}CH_2CH_2Cl$$